# EUROPÄISCHE PATENTSCHRIFT

(11) **EP 2 256 293 B1**
(45) Veröffentlichungstag und Bekanntmachung des Hinweises auf die Patenterteilung: **14.06.2017**
(21) Anmeldenummer: 09015847.8
(22) Anmeldetag: 22.12.2009
(51) Int. Cl.: F01C 1/14, A61B 17/16, A61C 1/05, F01C 1/08, F01C 13/02

(54) **Zahnradmotor und Verfahren zur Montage eines Zahnradmotors**
Gear motor and method for assembling same
Moteur à roue dentée et procédé de montage

(30) Priorität: 20.05.2009 DE 102009022121
(43) Veröffentlichungstag der Anmeldung: 01.12.2010
(73) Patentinhaber: WSEngineering GmbH & Co. KG, 78054 Villingen-Schwenningen (DE)
(72) Erfinder: Lay, Norbert, Dr., 78054 Villingen-Schwenningen (DE)
(74) Vertreter: Herden, Andreas F.

(56) Entgegenhaltungen:
- DE-B- 1 018 674
- DE-C- 547 176
- GB-A- 583 940
- GB-A- 1 000 999

## Beschreibung

### Gebiet der Erfindung

Die Erfindung betrifft einen Zahnradmotor, insbesondere einen Zahnradmotor zur Verwendung in chirurgischen und zahnärztlichen Instrumenten sowie eine Bohrspindel für den industriellen Einsatz.

### Hintergrund der Erfindung

Zahnradmotoren sind bekannt.

Bei medizinischen Geräten wie Knochenbohrern werden aber üblicherweise alternativ zu Elektromotoren druckluftbetriebene Turbinenmotoren oder Lamellenmotoren verwendet. Diese haben zumeist den Nachteil, dass sie einem hohen Verschleiß unterliegen und dass sie sich nur schlecht oder gar nicht sterilisieren lassen.

Die deutsche Gebrauchsmusterschrift DE 29802792 U1 (Aeskulap KG & Co.) schlägt als Antriebsmotor einen Zahnradmotor für medizinische Geräte vor.

Auch die Dokumente DE 547 176 A, DE 1 018 674 A und GB 1 000 999 B zeigen Zahnradmotoren.

Nachteilig an derartigen Zahnradmotoren hat sich gezeigt, dass die Leistung eines mit einem Zahnradmotor angetriebenen Gerätes oft nicht ausreichend ist. Weiter hat sich gezeigt, dass auch derartige Zahnradmotoren, insbesondere wenn sie bei hohen Drehzahlen betrieben werden, einem recht hohen Verschleiß unterliegen. Weiter sind bekannte Zahnradmotoren oft unangenehm laut, so dass eine Verwendung zumindest im zahnärztlichen Bereich nicht in Betracht kommt.

Insbesondere neigen bei bekannten Zahnradmotoren, bei denen die Zahnräder auf Kugellagern gelagert sind, die Kugellager zu einem hohen Verschleiß. So sind bei Drehzahlen von etwa 80000 U/min die Kugellager oft schon nach wenigen Betriebsstunden verschlissen.

### Aufgabe der Erfindung

Der Erfindung liegt demgegenüber die Aufgabe zugrunde, einen Zahnradmotor bereitzustellen, bei welchem die genannten Nachteile des Standes der Technik zumindest reduziert sind.

Es ist insbesondere Aufgabe der Erfindung, die Leistung des Zahnradmotors bei gleichbleibenden Abmessungen und gleichbleibendem Fluiddruck, insbesondere bei gleichbleibenden Abmessungen der Zahnräder gegenüber dem Stand der Technik zu erhöhen.

Weiter soll die Leistung über den gesamten Drehzahlbereich deutlich verbessert werden.
Eine weitere Aufgabe der Erfindung ist es, den Zahnradmotor möglichst leise laufend zu gestalten und den Verbrauch von Antriebsfluid zu minimieren.

### Zusammenfassung der Erfindung

Die Aufgabe der Erfindung wird bereits durch einen Zahnradmotor sowie ein Verfahren zur Montage eines Zahnradmotors nach einem der unabhängigen Ansprüche gelöst.

Bevorzugte Ausführungsformen und Weiterbildungen der Erfindung sind den jeweiligen Unteransprüchen zu entnehmen.

Die Erfindung betrifft zum Einen ein Verfahren zur Montage eines Zahnradmotores, wobei der Zahnradmotor zumindest einen auf zwei Wälzlagern gelagerten Rotor umfasst. Vorzugsweise umfasst der Zahnradmotor zwei Rotoren, von denen einer als Antriebswelle dient.

Gemäß der Erfindung wird der Außenring eines ersten Wälzlagers im Statorgehäuse befestigt, also derart fixiert, dass der Außenring des Wälzlagers sich nicht mehr zum Statorgehäuse bewegen kann.

Sodann wird der Innenring des ersten Wälzlagers und der Rotor mit einem vorgegebenen Drehmoment verschraubt. Unter einem vorgegebenen Drehmoment wird auch ein Drehmomentbereich verstanden.

Das Verschrauben des Innenrings des ersten Wälzlagers führt dazu, dass der Rotor an der Innenwand des Statorgehäuses zur Anlage kommt.

Sodann wir der Außenring des zweiten Wälzlagers, also des Wälzlagers auf der gegenüberliegenden Seite ebenfalls mit dem Statorgehäuse fest verbunden. Der erfindungsgemäße Motor weist somit zwei Festlager auf.

Danach wird der Innenring des zweiten Wälzlagers mit dem Rotor verschraubt, wobei die Verschraubung derart angezogen wird, dass der Rotor in axialer Richtung zentriert wird.

Die Erfindung ermöglicht so eine sehr exakte Positionierung des Rotors, wobei durch die resultierende Verspannung der Wälzlager, welche vorzugsweise als Kugellager ausgebildet sind, die Lage des Rotors zum Statorgehäuse mit sehr geringen Toleranzen eingestellt werden kann.

Aufgrund des geringen Spiels und aufgrund des möglichen geringen Abstands des Rotors zur Wand des Statorgehäuses an den Stirnseiten kann ein Zahnradmotor mit hoher Leistung bereitgestellt werden, da die Leckage minimiert werden kann.

Die Zentrierung des Rotors in axialer Richtung kann bei entsprechend geringen Bauteiltoleranzen über das Drehmoment der Verschraubung des zweiten Wälzlagers eingestellt werden.

Alternativ ist auch denkbar, über eine Messeinrichtung den Abstand der Stirnseiten des Rotors zum Statorgehäuse zu messen.

Bei einer bevorzugten Ausführungsform der Erfindung wird eine Distanzhülse zwischen den Innenring der Wälzlager und den Rotor gesetzt.

Über eine Distanzhülse kann mit höherer Präzision der Zwischenraum zwischen Wälzlager und stirnseitiger Statorwand verkleinert werden.

Weiter ist es denkbar, über die Distanzhülse Maßtoleranzen, insbesondere des Innenrings des Wälzlagers, auszugleichen.

Die Außenringe werden vorzugsweise in das Statorgehäuse eingepresst oder eingeklebt.

Bei einer bevorzugten Ausführungsform der Erfindung erfolgt die Verschraubung zumindest des Innenrings des zweiten Wälzlagers mit dem Rotor selbstsichernd. Hierzu kann beispielsweise die Schraube mit der Welle des Rotors verklebt werden.

Dies ist insbesondere von Vorteil, wenn die Verschraubung des zweitens Wälzlagers nicht mit einem vorgegebenen Drehmoment erfolgt, welches eine Sicherung der Schraube zur Folge hat, sondern das Anziehen der Schraube in erster Linie der axialen Positionierung des Rotors dient.

Die Erfindung betrifft des Weiteren einen Zahnradmotor, welcher einen Stator und zumindest einen Rotor umfasst, wobei der Rotor auf einer Welle angeordnet ist, die beidseitig auf Wälzlagern, insbesondere auf Kugellagern gelagert ist.

Gemäß der Erfindung ist der Außenring beider Wälzlager fest mit dem Statorgehäuse verbunden und auf der Welle zwischen dem Wälzlager und dem Rotor ist jeweils ein Distanzring angeordnet.

Es hat sich herausgestellt, dass die Verwendung zweier Festlager und eines Distanzrings, welcher den Zwischenraum zwischen der Stirnseite des Rotors und der stirnseitigen Gehäusewand des Stators verkleinert, die Leistung eines Zahnradmotors signifikant erhöht werden kann, da die Leckage verringert wird.

Des Weiteren konnte aufgrund des reduzierten Lagerspiels der Verschleiß, insbesondere des Zahnrads der Antriebswelle, reduziert werden. Gleichzeitig sinkt der Geräuschpegel des Zahnradmotors spürbar.

Es ist zu vermuten, dass dieser Effekt darauf zurückzuführen ist, dass bei bekannten Zahnradmotoren Antriebsfluid ungleichmäßig am vorderen und hinteren Teil des Zahnrads vorbeiströmt und dass dadurch das Zahnrad in Schwingungen kommt oder gegen die vordere Gehäuseplatte gedrückt wird.

Die Wälzlager sind axial nach innen gegeneinander verspannt, wodurch das Lagerspiel weiter reduziert wird. Der Innenring des Wälzlagers

Vorzugsweise werden die Wälzlager mit einer Kraft zwischen 2 und 50 N, besonders bevorzugt zwischen 5 und 15 N, gegeneinander verspannt.

Bei einer bevorzugten Ausführungsform der Erfindung werden die Außenringe der Wälzlager in das Statorgehäuse eingepresst oder eingeklebt.

So lässt sich ohne hohen Aufwand eine besonders exakte Position der Außenringe einstellen.

Durch die Erfindung kann ein Zahnradmotor bereitgestellt werden, bei dem das Axialspiel zumindest eines Zahnrades im Statorgehäuse weniger als 10/100, bevorzugt weniger als 5/100 und besonders bevorzugt weniger als 2/100 mm beträgt.

Der stirnseitige Abstand zumindest eines Zahnrads zum Statorgehäuse lässt sich auf zumindest einer Seite, bevorzugt auf beiden Seiten auf weniger als 20/100, bevorzugt weniger als 10/100 und besonders bevorzugt auf weniger als 5/100 einstellen.

Bei einer bevorzugten Ausführungsform der Erfindung ist der Zahnradmotor im Wesentlichen aus rostfreiem Stahl ausgebildet. So kann der Zahnradmotor, welcher insbesondere für medizinische Anwendungen vorgesehen ist, beispielsweise in einem Autoklaven desinfiziert werden.

Alternativ oder in Kombination kann auch Aluminium oder Kunststoff als Material, insbesondere für das Gehäuse, verwendet werden.

Leichte Materialien, wie Aluminium oder Kunststoff können auch zur Verringerung des Trägheitsmomentes der Rotoren verwendet werden. Insbesondere sind Rotoren mit einer Achse aus Metall und Zähnen aus Kunststoff vorgesehen. So kann ein Nachlaufen des Zahnradmotors nach Schließen der Luftzufuhr, welches in machen medizinischen Anwendungen unerwünscht ist, deutlich verringert werden.

Bei einer Weiterbildung der Erfindung sind die Wälzlager als Keramikkugellager ausgebildet, welche einem noch geringeren Verschleiß als Stahlkugellager unterliegen. Derartige Kugellager werden vorzugsweise mit einer geringen Restschmierung betrieben und können daher selbst eine Desinfektion in einem Autoklaven oder einem Wasserbad überstehen, ohne danach mit einem Schmiermittel behandelt werden zu müssen.

Der erfindungsgemäße Zahnradmotor hat vorzugsweise bei einem Fluiddruck des Antriebsfluids von 6 bis 8 Bar eine Leistung zwischen 100 und 450 W, bevorzugt zwischen 150 und 300 W.

Die derartig hohe Leistung bewirkt ein maximales Drehmoment über den gesamten Drehzahlbereich, insbesondere erzielt der Motor ein maximales Drehmoment im niedrigen Drehzahlbereich.

Bei einer bevorzugten Ausführungsform der Erfindung beträgt das Verhältnis von Länge zu Breite der Zahnräder zwischen 1 und 4, bevorzugt zwischen 2 und 3. Insbesondere beträgt das Verhältnis etwa 2,1.

Bei einer Weiterbildung der Erfindung sind die Zahnräder mit einem Kunststoff beschichtet oder bestehen im Wesentlichen aus Kunststoff.

Hierfür ist insbesondere Polytetrafluorethylen (PTFE) oder Polyetheretherkethon (PEEK) geeignet. Durch eine derartige Beschichtung kann nicht nur die Lebensdauer der Zahnräder durch die selbstschmierenden Eigenschaften des Werkstoffes erhöht werden, sondern vor allem kann der Geräuschpegel des Zahnradmotors abgesenkt werden.

Die Beschichtung ist dabei vorzugsweise bis mindestens 100° C, bevorzugt bis 140° C temperaturstabil, so dass der Zahnradmotor zur Desinfektion autoklaviert werden kann.

Bei einer Weiterbildung der Erfindung ist der Zahnradmotor mit Ausnahme der Antriebswelle vollständig gekapselt. Insbesondere die Lager des Motors liegen so nicht frei und der Motor besitzt eine hohe Dichtigkeit. So ist der Motor vor allem für medizinische Anwendungen gut geeignet.

Die Erfindung bezieht sich vorzugsweise auf Motoren mit relativ kleinen Zahnrädern mit einem Außendurchmesser der Zahnräder zwischen 5 und 35 mm, vorzugsweise zwischen 7 und 18 mm. Dabei haben die Zahnräder zwischen 5 und 21, vorzugsweise zwischen 7 und 13 Zähne.

Bei einer Weiterbildung der Erfindung weist der Zahnradmotor Rotoren mit einer unterschiedlichen Anzahl an Zähnen auf. Beispielsweise kann ein Rotor mit 10 und der andere Rotor mit 11 Zähnen versehen sein. Es hat sich herausgestellt, dass so der Geräuschpegel des Zahnradmotors reduziert werden kann, insbesondere können Peaks im Bereich hoher Frequenzen vermieden werden.

Vorzugsweise umfasst der Zahnradmotor zumindest zwei Rotoren und ein Statorgehäuse. Bevorzugt weist das Statorgehäuse an der Stirnseite der Rotoren zumindest eine Ausnehmung auf, in welche zwischen den Rotoren komprimierte Luft einströmen kann.

So lässt sich so die Geräuschentwicklung eines Zahnradmotors erheblich reduzieren. Es ist zu vermuten, dass zwischen den Zähnen der Rotoren befindliche Luft, welche durch das Ineinandergreifen der Zahnräder komprimiert wird, in hohem Maße zur Geräuschentwicklung des Motors beiträgt. Weiter wird ein Pulsieren der Lager weitgehend vermieden und so die Beanspruchung der Lager verringert.

Über eine Ausnehmung wird erreicht, dass die zwischen den Zähnen komprimierte Luft entweichen kann. Die Ausnehmung kann als bloßes Puffervolumen ausgebildet sein oder, wie es bei einer bevorzugten Ausführungsform der Erfindung vorgesehen ist, den Bereich, in welchem sich die Rotoren überschneiden, mit der Auslassseite des Zahnradmotors verbinden. Hierzu kann die Ausnehmung insbesondere auf der Luftaustrittsseite des Zahnradmotors mit einer Kammer des Statorgehäuses verbunden sein. Mit einer einlassseitigen Kammer des Statorgehäuses ist die Ausnehmung dagegen nicht verbunden, da ansonsten Fluid durch die Kammer an den Rotoren vorbeiströmen könnte.

Vorzugsweise ist die Ausnehmung als Kanal ausgebildet, welcher im Wesentlichen mittig zwischen den Rotoren von einem Überschneidungsbereich der Rotoren bis in eine luftaustrittsseitige Kammer des Statorgehäuses läuft.

Die Breite der Ausnehmung liegt bei einer bevorzugten Ausführungsform zwischen dem 0,5 und 2fachen der maximalen Breite des Bereiches in dem sich die Rotoren mit ihren Zähnen überschneiden.

Um eine hinreichend schnelle Strömung der komprimierten Luft aus dem Zwischenraum zwischen den Rotoren erreichen zu können, hat die Ausnehmung bei einer bevorzugten Ausführungsform der Erfindung eine Tiefe von mindestens 0,5, besonders bevorzugt mindestens 1 mm.

Der erfindungsgemäße Zahnradmotor ist für chirurgische und zahnärztliche Instrumente, wie Knochenbohrer, Zahnbohrer etc. vorgesehen.

Eine alternative Ausführungsform, bei der insbesondere auf korrosionsresistente und hitzebeständige Materialien verzichtet werden kann, eignet sich auch mit entsprechendem Getriebe für Bohrwerkzeuge im Bereich des Maschinenbaus.

### Kurzbeschreibung der Zeichnungen

Die Erfindung soll im Folgenden bezugnehmend auf ein schematisch dargestelltes Ausführungsbeispiel anhand der Zeichnungen Fig. 1 bis Fig. 5 näher erläutert werden.
- Fig. 1: zeigt eine schematische Schnittansicht eines Zahnradmotors,
- Fig. 2: zeigt schematisch eine perspektivische Ansicht auf die Rückseite eines Zahnradmotors,
- Fig. 3: zeigt schematisch eine Schnittansicht entlang einer Ebene, die senkrecht zur Achse des Zahnradmotors verläuft,
- Fig. 4: zeigt eine Ansicht auf die Vorderseite des Zahnradmotors,
- Fig. 5: zeigt schematisch die Verspannung der Kugellager,
- Fig. 6 und 7: zeigen schematische isometrische Ansichten einer vorderen und hinteren Lagerplatte, welche zur Geräuschreduzierung mit einer Ausnehmung versehen sind.

### Detaillierte Beschreibung der Zeichnungen

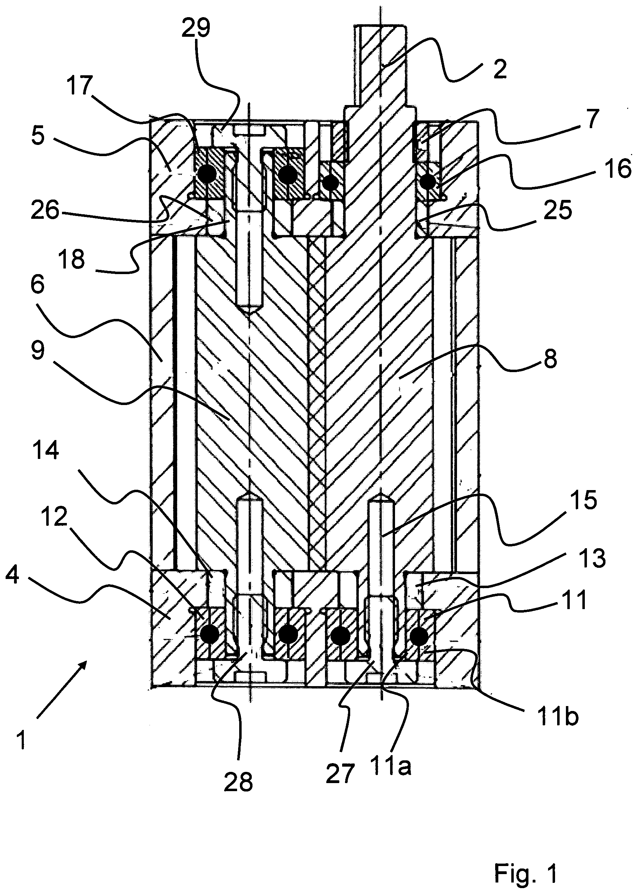

Fig. 1 zeigt eine schematische Schnittansicht eines Zahnradmotors 1 entlang der Achsen der Rotoren.

Der Zahnradmotor 1 umfasst ein Gehäuse, welches eine hintere Lagerplatte 4, eine vordere Lagerplatte 5 und einen Statorabschnitt 6 umfasst. Der Statorabschnitt 6 befindet sich zwischen hinterer 4 und vorderer 5 Lagerplatte und ist mit dem Lagerplatten 4, 5 verschraubt.

Im Statorabschnitt 6 laufen die Rotoren, welche als Zahnräder 8, 9 ausgebildet sind. Es kann sich dabei beispielsweise um Zahnräder mit einer Evolventenverzahnung handeln.

Das Zahnrad 8 dient dem Antrieb der Antriebswelle 2.

Die Zahnräder 8, 9 sind jeweils auf einer Welle 18 angeordnet. In diesem Ausführungsbeispiel ist die Welle 18 mit den Zahnrädern 8, 9 als einstückiges Bauteil ausgebildet.

Die Zahnräder 8, 9 sind über die Kugellager 11, 12 und 16, 17 drehbar gelagert.

Details werden im Folgenden Bezug nehmend auf das Kugellager 11 dargestellt.

Der Außenring 11b des Kugellagers 11, sowie entsprechend auch die Außenringe der Kugellager 12, 16 und 17, ist in dem Statorgehäuse eingepresst oder eingeklebt. Die Lager 11, 12, 16 und 17 sind also als Festlager ausgebildet.

Der Innenring 11a des Kugellagers 11, beziehungsweise die Innenringe der Kugellager 12 und 17 sind jeweils mit der Welle der Zahnräder 8, 9 über die Schrauben 27, 28 und 29 verschraubt. Beim Kugellager 16 erfolgt die Verschraubung nicht über eine Schraube, sondern über eine Mutter 7, welche auf der Antriebswelle 2 sitzt.

Die Montage des Zahnradmotors kann beispielsweise wie folgt erfolgen.

Zunächst werden die Zahnräder 8, 9 in den Statorabschnitt 6 eingebracht und das Gehäuse bestehend aus vorderer 5 und hinterer 4 Lagerplatte sowie Statorabschnitt 6 zusammengeschraubt.

Sodann wir das Kugellager 11 in die hintere Lagerplatte 4 eingepresst oder eingeklebt.

Dann wird der Rotor 8 mittels der Schraube 27 mit einem vorgegebenen Drehmoment mit dem Innenring 11a des Kugellagers 11 verschraubt. Dabei kommt der Rotor 8 an der hinteren Gehäuseplatte 4 zur Anlage.

Sodann wir das Kugellager 16 in der vorderen Gehäuseplatte 5 eingepresst oder eingeklebt.

Über die Mutter 7 wird sodann der Innenring des Kugellagers 16 mit der Antriebswelle 2 verbunden.

Dabei wird die Mutter 7 solange angezogen, bis das Zahnrad 8 in axialer Richtung zentriert ist. Die Kugellager 11 und 16 sind gegeneinander verspannt und es ist durch die axiale Zentrierung möglich, den Spalt zwischen dem Zahnrad 8 sowie vorderer 5 und hinterer 4 Gehäuseplatte sehr klein zu halten.

Die Montage des zweiten Zahnrads 9 erfolgt entsprechend.

Weiter umfasst der Zahnradmotor die Distanzringe 13, 14 und 25, 26, welche jeweils zwischen dem Innenring der Kugellager 11, 12, 16 und 17 und den Rotoren 8, 9 angeordnet sind.

Über die Distanzringe 13, 14, 25, 26 wird der Luftspalt zwischen den Stirnseiten der Zahnräder 8, 9 und den Gehäuseplatten 4, 5 weiter verkleinert. Auch eine bessere Montage wird ermöglicht, da die Distanz zumindest von einer Seite vorgegeben ist.

Auch ist es denkbar, mittels der Distanzringe mögliche Fertigungstoleranzen, insbesondere Fertigungstoleranzen der Kugellager auszugleichen.

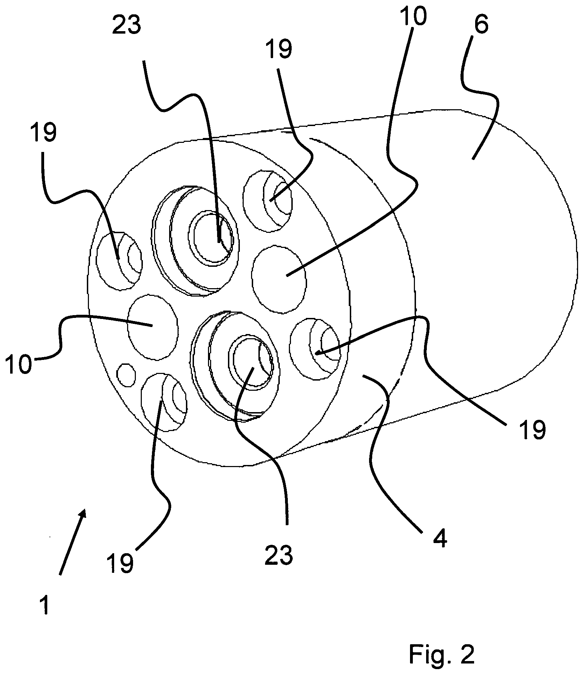

Fig. 2 zeigt schematisch eine perspektivische Ansicht eines Zahnradmotors 1 von der Rückseite. Die Rückseite 1 umfasst zwei Kanäle 10, über die das Antriebsfluid in den Zahnradmotor 1 ein- und wieder ausströmt. Weiter sind hier in der hinteren Abschlussplatte 4 rückseitige Bohrungen 23 zu erkennen, in welchen die Wellen der Zahnräder (nicht dargestellt) zusammen mit den Kugellagern (nicht dargestellt) in der hinteren Lagerplatte 4 eingepresst oder eingeklebt sind. Die Bohrungen 23 bilden dabei einen Anschlag für die Außenringe der Kugellager (nicht darstellt), wodurch deren Position im Gehäuse definiert wird. Um die hintere Abschlussplatte 4 mit dem Statorabschnitt 6 zu verbinden, weist die hintere Abschlussplatte 4 Bohrungen 19 auf, durch die Schrauben (nicht dargestellt) geführt werden, mit denen die hintere Abschlussplatte am Statorabschnitt 6 verschraubt wird.

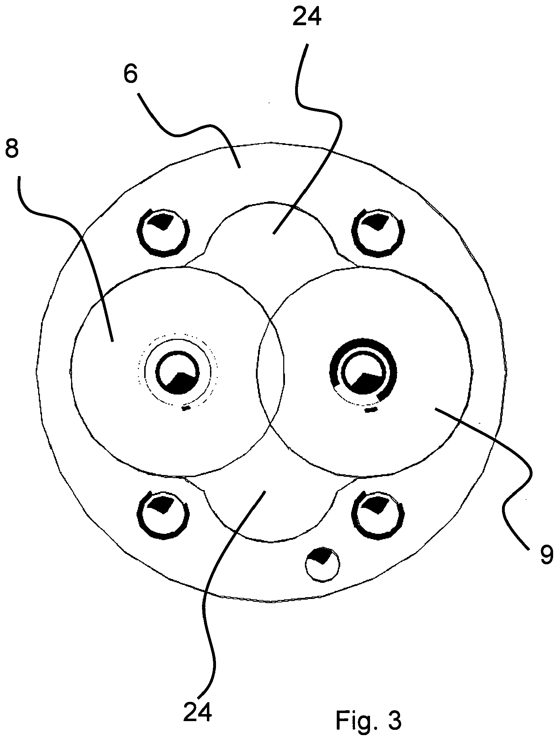

Fig. 3 zeigt schematisch eine Schnittansicht eines Zahnradmotors parallel zur Stirnseite.

Zu erkennen sind die beiden Zahnräder 8, 9, welche im mittleren Statorabschnitt 6 laufen.

Beidseitig umfasst der mittlere Statorabschnitt 6 jeweils eine Kammer 24, in die über Kanäle (nicht dargestellt) das Antriebsfluid ein- und ausgeleitet wird. Je nachdem, auf welcher Seite der Fluidanschluss angeordnet wird, kann der Zahnradmotor in beiden Richtungen betrieben werden.

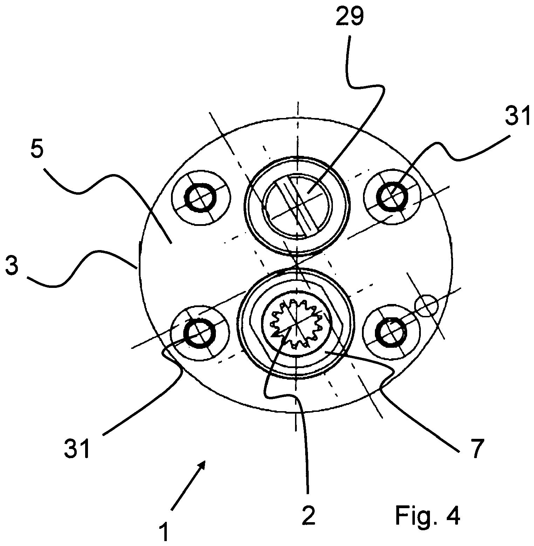

Fig. 4 zeigt schematisch eine Ansicht auf die vordere Gehäuseplatte 5 des Zahnradmotors 1.

Das Gehäuse 3 des Zahnradmotors 1 ist im Wesentlichen kreiszylinderförmig ausgebildet.

Aus der vorderen Lagerplatte 5 ragt die Antriebswelle 2 hervor.

Zu erkennen ist ferner die Schraube 29, mittels der eines der Zahnräder befestigt ist. Das Zahnrad, welches die Antriebswelle 2 aufweist, ist mit der Mutter 7 befestigt. Weiter weist die vordere Lagerplatte 5 Bohrungen 31 auf, über die die vordere Lagerplatte 5 mittels Schrauben (nicht dargestellt) mit dem Statorabschnitt (nicht dargestellt) verschraubt wird.

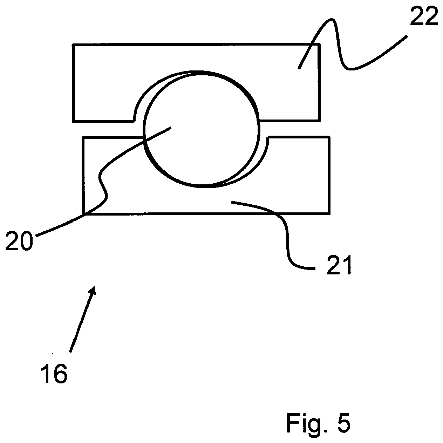

Bezugnehmend auf Fig. 5 soll die Verspannung eines Kugellagers näher erläutert werden. Schematisch dargestellt ist ein Kugellager 16 in einer Schnittansicht, welches verspannt eingebaut ist. Dabei ist der Innenring 21 des Kugellagers 16 zu dem Außenring 22 in axialer Richtung leicht verschoben, wodurch die Kugel 20 ihre Vorzugsstellung nicht mehr genau auf einer Nullposition, die durch die Mitte der Lagerschale definiert wird hat, sondern von dieser Mittelposition versetzt hat. Es hat sich herausgestellt, dass so die Lebensdauer der Kugellager signifikant erhöht wird. Des weiteren werden Toleranzen der Kugeln und Lagerschalen eliminiert, die Kugeln bewegen sich nicht in den Lagern in axialer Richtung.

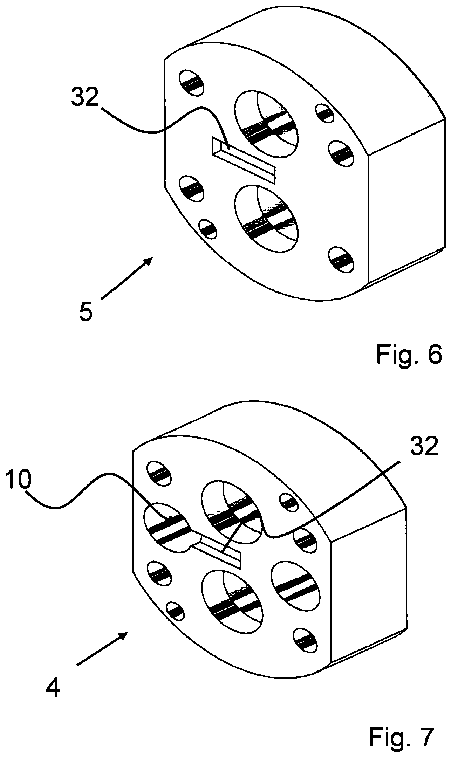

Bezugnehmend auf Fig. 6 und Fig. 7 soll eine weitere Erfindung näher erläutert werden, bei der über eine Ausnehmung 32 die Geräuschemission sowie die Belastung der Lager des Zahnradmotors reduziert werden.

In Fig. 6 ist eine vordere Lagerplatte 5 dargestellt, welche ansonsten den zuvor beschriebenen Lagerplatten entsprechen kann.

Die in Fig. 6 dargestellte Lageplatte, welche ein Teil des Statorgehäuses (nicht dargestellt) bildet, weist eine stirnseitig vor den Rotoren (nicht dargestellt) angeordnete Ausnehmung 32 auf.

Die Ausnehmung 32 ist als im Wesentlichen nutförmiger Kanal ausgebildet und verläuft vom Überschneidungsbereich der Rotoren (nicht dargestellt) bis in die auslassseitige Kammer (nicht dargestellt) des Statorgehäuses.

Antriebsfluid, welches zwischen den Zähnen der Rotoren komprimiert wird, kann durch die Ausnehmung 32 in die auslasseitige Kammer (24 in Fig. 3) des Statorgehäuses entweichen. Die Ausnehmung 32 ist allerdings nur auf einer Seite mit der Kammer des Statorgehäuses verbunden, um zu verhindern, dass Antriebsfluid durch die Ausnehmung 32 unkontrolliert von einer Kammer in die andere fließt. Vorzugsweise ist die Ausnehmung mit der auslassseitigen Kammer des Statorgehäuses verbunden, um das Antriebsfluid über den Auslass des Zahnradmotors abzuführen.

Fig. 7 zeigt, schematisch dargestellt, eine hintere Lagerplatte 4, bei welcher ebenfalls eine entsprechende Ausnehmung 32 vorhanden ist.

Die Ausnehmung 32 führt hier direkt in den Kanal 10, über welchen das Antriebsfluid den Zahnradmotor verlässt.

Es versteht sich, dass die Erfindung nicht auf eine Kombination vorstehend beschriebener Merkmal beschränkt ist, sondern dass der Fachmann sämtliche beschriebenen Merkmale, soweit dies sinnvoll ist, kombinieren wird.

### Bezugszeichenliste

- 1: Zahnradmotor
- 2: Antriebswelle
- 3: Gehäuse
- 4: hintere Lagerplatte
- 5: vordere Lagerplatte
- 6: Statorabschnitt
- 7: Mutter
- 8: Zahnrad
- 9: Zahnrad
- 10: Kanal
- 11: Kugellager
- 12: Kugellager
- 13: Distanzring
- 14: Distanzring
- 15: Gewinde
- 16: Kugellager
- 17: Kugellager
- 18: Welle
- 19: Bohrung
- 20: Kugel
- 21: Innenring
- 22: Außenring
- 23: Bohrung
- 24: Kammer
- 25: Distanzring
- 26: Distanzring
- 27: Schraube
- 28: Schraube
- 29: Schraube
- 30: Schraube
- 31: Bohrung
- 32: Ausnehmung

## Patentansprüche

1. Verfahren zur Montage eines Zahnradmotors (1), welcher zumindest einen auf zwei Wälzlagern gelagerten Rotor umfasst, umfassend die Schritte:
- Befestigen des Außenrings (22) des ersten Wälzlagers im Statorgehäuse,
- Verschrauben des Innenrings (21) des ersten Wälzlagers und des Rotors mit einem vorgegebenen Drehmoment,
- Befestigen des Außenrings (22) des zweiten Wälzlagers im Statorgehäuse,
- Verschrauben des Innenrings (21) des zweiten Wälzlagers und des Rotors, wobei die Verschraubung derart angezogen wird, dass der Rotor in axialer Richtung zentriert wird.

2. Verfahren zur Montage eines Zahnradmotors nach dem vorstehenden Anspruch, **dadurch gekennzeichnet, dass** ein Distanzring (13,14) zwischen Innenring (21) und Rotor gesetzt wird.

3. Verfahren zur Montage eines Zahnradmotors nach einem der vorstehenden Ansprüche, **dadurch gekennzeichnet, dass** die Wälzlager axial gegeneinander verspannt werden.

4. Verfahren zur Montage eines Zahnradmotors nach einem der vorstehenden Ansprüche, **dadurch gekennzeichnet, dass** die Verschraubung zumindest des Innenrings (21) des zweiten Wälzlagers mit dem Rotor selbstsichernd ausgeführt wird.

5. Zahnradmotor (1), umfassend einen Stator und zumindest einen Rotor, wobei der Rotor auf einer Welle angeordnet ist, die beidseitig auf einem ersten und einem zweiten Wälzlager gelagert ist,
wobei der Außenring (22) beider Wälzlager (11,16) fest mit dem Statorgehäuse verbunden ist,
und wobei zumindest auf der Welle zwischen dem zweiten Wälzlager und dem Rotor ein Distanzring (13) angeordnet ist, **dadurch gekennzeichnet, dass** die Wälzlager axial nach innen gegeneinander verspannt sind und dass der Innenring (21) zumindest des zweiten Wälzlagers mit dem Rotor verschraubt ist.

6. Zahnradmotor (1) nach dem vorstehenden Anspruch, **dadurch gekennzeichnet, dass** die Wälzlager als Kugellager (11,12,16,17) ausgebildet sind.

7. Zahnradmotor (1) nach einem der vorstehenden Ansprüche, **dadurch gekennzeichnet, dass** die Außenringe (22) der Wälzlager in das Statorgehäuse eingepresst und/oder eingeklebt ist.

8. Zahnradmotor (1) nach einem der vorstehenden Ansprüche, **dadurch gekennzeichnet, dass** das Statorgehäuse eine hintere Lagerplatte (4), einen mittleren Statorabschnitt (6) und eine vorderen Lagerplatte (5) aufweist, welche miteinander verbunden sind.

9. Zahnradmotor (1) nach einem der vorstehenden Ansprüche, **dadurch gekennzeichnet, dass** der stirnseitige Abstand zumindest eines Zahnrades (8,9) zum Statorgehäuse auf zumindest einer Seite, vorzugsweise auf beiden Seiten weniger als 20/100, bevorzugt weniger als 10/100 und besonders bevorzugt weniger als 5/100 mm beträgt.

10. Zahnradmotor (1) nach einem der vorstehenden Ansprüche, **dadurch gekennzeichnet, dass** das Verhältnis von Länge zu Breite der Zahnräder (8,9) zwischen 1 und 4, bevorzugt zwischen 2 und 3 liegt.

11. Zahnradmotor (1) nach einem der vorstehenden Ansprüche, **dadurch gekennzeichnet, dass** die Zahnräder (8,9) mit einem Kunststoff beschichtet sind oder aus Kunststoff bestehen.

12. Zahnradmotor (1) nach einem der vorstehenden Ansprüche, **dadurch gekennzeichnet, dass** die Rotoren eine unterschiedliche Anzahl an Zähnen aufweisen.

13. Zahnradmotor (1) nach einem der vorstehenden Ansprüche, **dadurch gekennzeichnet, dass** das Statorgehäuse an der Stirnseite der Rotoren zumindest eine Ausnehmung (32) aufweist, in die zwischen den Rotoren komprimierte Luft einströmen kann.

14. Zahnradmotor (1) nach dem vorstehenden Anspruch, **dadurch gekennzeichnet, dass** die Ausnehmung (32) auf der Luftaustrittsseite des Zahnradmotors mit einer Kammer (24) des Statorgehäuses verbunden.

15. Zahnradmotor nach einem der vorstehenden beiden Ansprüche, **dadurch gekennzeichnet, dass** die Ausnehmung (32) als Kanal ausgebildet ist, welcher sich mittig zwischen den Rotoren von einem Überschneidungsbereich der Rotoren bis in eine luftaustrittsseitige Kammer des Statorgehäuses erstreckt.

## Claims

1. A method for assembling a gear motor (1) which comprises at least one rotor mounted on two rolling bearings, comprising the steps of:
- fastening the outer ring (22) of the first rolling bearing in the stator housing;
- screwing together, with a predetermined torque, the inner ring (21) of the first rolling bearing and the rotor;
- fastening the outer ring (22) of the second rolling bearing in the stator housing;
- screwing together the inner ring (21) of the second rolling bearing and the rotor, wherein the screw connection is tightened so that the rotor is centered in axial direction.

2. The method for assembling a gear motor according to the preceding claim, **characterized in that** a spacer ring (13, 14) is placed between the inner ring (21) and the rotor.

3. The method for assembling a gear motor according to any one of the preceding claims, **characterized in that** the rolling bearings are braced axially against each other.

4. The method for assembling a gear motor according to any one of the preceding claims, **characterized in that** the screw connection of at least the inner ring (21) of the second rolling bearing with the rotor is effected in self-locking manner.

5. A gear motor (1), comprising a stator and at least one rotor, wherein the rotor is arranged on a shaft which is supported on both ends thereof on first and second rolling bearings,
wherein the outer ring (22) of both rolling bearings (11, 16) is fixedly connected to the stator housing, and wherein a spacer ring (13) is arranged at least on the shaft between the second rolling bearing and the rotor, **characterized in that** the rolling bearings are braced axially inwardly against each other, and that the inner ring (21) of at least the second rolling bearing is screwed to the rotor.

6. The gear motor (1) according to the preceding claim, **characterized in that** the rolling bearings are ball bearings (11, 12, 16, 17).

7. The gear motor (1) according to any one of the preceding claims, **characterized in that** the outer rings (22) of the rolling bearings are pressed and/or glued into the stator housing.

8. The gear motor (1) according to any one of the preceding claims, **characterized in that** the stator housing comprises a rear bearing plate (4), a central stator portion (6), and a front bearing plate (5) which are connected to one another.

9. The gear motor (1) according to any one of the preceding claims, **characterized in that** the spacing between an end face of at least one gear (8, 9) and the stator housing is less than 20/100, preferably less than 10/100, and more preferably less than 5/100 mm, at least on one side, preferably on both sides.

10. The gear motor (1) according to any one of the preceding claims, **characterized in that** the ratio of length to width of the gears (8, 9) is between 1 and 4, preferably between 2 and 3.

11. The gear motor (1) according to any one of the preceding claims, **characterized in that** the gears (8, 9) are coated with a plastic or consist of plastic.

12. The gear motor (1) according to any one of the preceding claims, **characterized in that** the rotors have a different number of teeth.

13. The gear motor (1) according to any one of the preceding claims, **characterized in that** the stator housing has at least one recess (32) at the end face of the rotors, into which air compressed between the rotors can flow.

14. The gear motor (1) according to the preceding claim, **characterized in that** the recess (32) on the air outlet side of the gear motor is in communication with a chamber (24) of the stator housing.

15. The gear motor according to any one of the two preceding claims, **characterized in that** the recess (32) is formed as a channel which extends centrally between the rotors from an area of overlap of the rotors into a chamber on the air outlet side of the stator housing.

## Revendications

1. Procédé de montage d'un moteur à engrenage (1), lequel comprend au moins un rotor monté sur deux paliers à roulement, comprenant les étampes :
- de fixation de la bague extérieure (22) du premier palier à roulement dans le logement de stator,
- de vissage de la bague intérieure (21) du premier palier à roulement et du rotor avec un couple prédéfini,
- de fixation de la bague extérieure (22) du deuxième palier à roulement dans le logement de stator,
- de vissage de la bague intérieure (21) du deuxième palier à roulement et du rotor, le vissage étant serré de telle sorte que le rotor est centré dans la direction axiale.

2. Procédé de montage d'un moteur à engrenage selon la revendication précédente, **caractérisé en ce qu'**une bague d'écartement (13, 14) est posée entre la bague intérieure (21) et le rotor.

3. Procédé de montage d'un moteur à engrenage selon l'une quelconque des revendications précédentes, **caractérisé en ce que** les paliers à roulement sont précontraints axialement les uns contre les autres.

4. Procédé de montage d'un moteur à engrenage selon l'une quelconque des revendications précédentes, **caractérisé en ce que** le vissage au moins de la bague intérieure (21) du deuxième palier à roulement au rotor est réalisé de manière autobloquante.

5. Moteur à engrenage (1), comprenant un stator et au moins un rotor, dans lequel le rotor est agencé sur un arbre qui est monté bilatéralement sur un premier et sur un deuxième palier à roulement,
dans lequel la bague extérieure (22) des deux paliers à roulement (11, 16) est reliée solidement au logement de stator,
et dans lequel une bague d'écartement (13) est agencée au moins sur l'arbre entre le deuxième palier à roulement et le rotor, **caractérisé en ce que** les paliers à roulement sont précontraints axialement vers l'intérieur les uns contre les autres et **en ce que** la bague intérieure (21) au moins du deuxième palier à roulement est vissée au rotor.

6. Moteur à engrenage (1) selon l'une quelconque des revendications précédentes, **caractérisé en ce que** les paliers à roulement sont réalisés sous la forme de roulements à billes (11, 12, 16, 17).

7. Moteur à engrenage (1) selon l'une quelconque des revendications précédentes, **caractérisé en ce que** les bagues extérieures (22) des paliers à roulements sont insérées et/ou collées dans le logement de stator.

8. Moteur à engrenage (1) selon l'une quelconque des revendications précédentes, **caractérisé en ce que** le logement de stator comprend une plaque de support arrière (4), une partie centrale de stator (6) et une plaque de support avant (5), lesquelles sont reliées les unes aux autres.

9. Moteur à engrenage (1) selon l'une quelconque des revendications précédentes, **caractérisé en ce que** l'écart frontal entre au moins une roue dentée (8, 9) et le logement de stator sur au moins une face, de préférence sur les deux faces, est inférieur à 20/100, de préférence inférieur à 10/100 et de manière particulièrement préférée inférieur à 5/100 mm.

10. Moteur à engrenage (1) selon l'une quelconque des revendications précédentes, **caractérisé en ce que** le rapport entre la longueur et la largeur des roues dentées (8, 9) est compris entre 1 et 4, de préférence entre 2 et 3.

11. Moteur à engrenage (1) selon l'une quelconque des revendications précédentes, **caractérisé en ce que** les roues dentées (8, 9) sont revêtues d'une matière plastique ou sont constituées d'une matière plastique.

12. Moteur à engrenage (1) selon l'une quelconque des revendications précédentes, **caractérisé en ce que** les rotors présentent un nombre différent de dents.

13. Moteur à engrenage (1) selon l'une quelconque des revendications précédentes, **caractérisé en ce que** le logement de stator comprend sur la face frontale des rotors au moins un évidement (32) dans lequel peut circuler l'air comprimé entre les rotors.

14. Moteur à engrenage (1) selon l'une quelconque des revendications précédentes, **caractérisé en ce que** l'évidement (32) sur la face de sortie d'air du moteur à engrenage est relié à une chambre (24) du logement de stator.

15. Moteur à engrenage (1) selon l'une quelconque des revendications précédentes, **caractérisé en ce que** l'évidement (32) est réalisé sous la forme d'un canal, lequel s'étend de manière centrale entre les rotors depuis une zone de chevauchement des rotors jusque dans une chambre côté sortie d'air du logement de stator.
